# EUROPEAN PATENT APPLICATION

(11) **EP 3 342 381 A1**
(43) Date of publication of application: **04.07.2018**
(21) Application number: 15902339.9
(22) Date of filing: 30.09.2015
(51) Int. Cl.: A61F 9/008

(54) **INCISION PORTION STRUCTURE, HAVING REINFORCEMENT PORTIONS TO BE FORMED ON CORNEA, FOR REMOVING LENTICULE INCISED ACCORDING TO EYE SIGHT CORRECTION SURGERY**

(30) Priority: 26.08.2015 KR 20150120141
(71) Applicant: Jung, Young Taek, Jeonju-si, Jeollabuk-do 54997 (KR)
(72) Inventor: Jung, Young Taek, Jeonju-si, Jeollabuk-do 54997 (KR)
(74) Representative: Dilg, Haeusler, Schindelmann Patentanwaltsgesellschaft mbH
(86) International application number: PCT/KR2015/010269
(87) International publication number: WO 2017/034067

(57) **Abstract**

Disclosed herein is an incision part formed in a cornea to remove a lenticule cut in the course of vision correction surgery, the vision correction surgery for correcting abnormal refraction being performed by drawing a dense spiral toward the center of the cornea or outward from the center of the cornea while three-dimensionally changing the focal point of a laser beam to form a flap surface and a lenticular surface each having a continuous spiral incision line in a corneal stroma such that the flap surface and the lenticular surface are spaced apart from each other, making the outer circumference of the lenticular surface intersect the outer circumference of the flap surface inside the outer circumference of the flap surface to cut a three-dimensional lenticule from the corneal stroma, and removing the lenticule out of the cornea through an incision part formed in the cornea, wherein the incision part includes an incision entrance formed in the surface of the cornea adjacent to the outer circumference of the flap surface, the incision entrance being incised by the laser beam, and an incision tunnel extending from the incision entrance toward the corneal stroma, the tip of the incision tunnel being connected to the flap surface, and wherein the tip of the incision tunnel is connected to an incision line of the flap surface, and reinforcement parts, which are regions to which no laser beam is applied, are formed on opposite sides of the tip of the incision tunnel in the incision line of the flap surface.

## Description

### [Technical Field]

The present invention relates to an incision part having a reinforcement part formed in a cornea to remove a lenticule cut in the course of vision correction surgery, which is performed to cut a corneal stroma in accordance with a desired amount of correction through the surface of the cornea to separate a piece of the corneal stroma, i.e. a lenticule, from the corneal stroma, wherein the size of the incision part, formed in the cornea to remove the separated lenticule, is minimized.

### [Background Art]

The background art is provided only for easy understanding of the preset invention, and therefore it should be noted that the background art is not regarded as the prior art.

In general, a cornea is located at the outermost surface of an eyeball. The cornea is a part of the eye through which light is transmitted first. The cornea is transparent. The cornea is a tissue in front of the eye, and refracts light to form an image on a retina. The retina is a tissue in the rear of the eye, and senses light from the image formed thereon and transmits a signal based on the image to the brain.

The surface of the cornea is kept moisturized by tears. The tears serve to uniformly transmit and refract light. As a result, a normal person may have a corrected eyesight of 1.0 or higher.

Since the surface of the cornea is an epithelium, like the skin, cells are uniformly arranged on the surface of the cornea even though the surface of the cornea is slightly rough. The surface of the cornea includes five or six layers. The cornea includes a corneal epithelium, a Bowman's membrane, a corneal stroma, and a corneal endothelium, arranged in order from the outside.

The corneal epithelium includes five or six layers. The corneal epithelium is connected to the epithelium of a conjunctiva. The corneal epithelium is pushed outward from the basal cell layer while gradually becoming flat, and is removed in seven days. When damaged, the corneal epithelium is regenerated rapidly.

The Bowman's membrane is a membrane including colorless transparent collagen fibers without cells. The Bowman's membrane is regarded as a modified portion of the corneal stroma. Once damaged, the Bowman's membrane is not regenerated, but leaves a scar.

The corneal stroma is a connective tissue that occupies about 90 % of the thickness of the cornea. The corneal stroma includes a type I collagen fiber having about 200 layers. The size and the direction of the corneal stroma are uniform, and therefore the corneal stroma is transparent.

A Descemet's membrane is the lowermost membrane of the corneal endothelium. The Descemet's membrane includes cells having three or four layers.

The corneal endothelium includes a single layer of flat hexagonal cells. The corneal endothelium plays an important role in maintaining the state of hydration of the cornea. When damaged, the corneal endothelium is not regenerated, unlike the corneal epithelium.

Meanwhile, vision correction surgery for changing the thickness of the cornea of the eyeball to correct imperfect vision of the eyeball is used as a method of correcting imperfect vision of the eyeball. Representative examples of vision correction surgery include laser in-situ keratomileusis (LASIK), laser assisted sub-epithelial keratomileusis (LASEK), and small incision lenticule extraction (SMILE).

Laser in-situ keratomileusis (LASIK) surgery is surgery for forming a corneal flap and then applying a laser beam to a corneal stroma to correct vision. Conventional photorefractive keratectomy (PRK), which is also called excimer laser surgery, has disadvantages in that the pain after surgical operation is severe, the cut region becomes turbid, and the time taken for vision recovery is about 6 months, which is relatively long. LASIK surgery solves the disadvantages of PRK surgery. In LASIK surgery, the front part of the cornea, including the corneal epithelium and the Bowman's membrane, is incised using a micro-scale cornea cutting knife or a laser to form a corneal flap, the corneal flap is bent backward, a laser beam is applied to the corneal stroma in accordance with a desired amount of correction, set through an inspection before the surgical operation, to cut the cornea, and then the corneal flap is returned to the original position thereof. LASIK surgery is vision correction surgery that is capable of reducing pain and turbidity of the cornea and shortening the vision recovery time.

Laser assisted sub-epithelial keratomileusis (LASEK) surgery is surgery for forming a corneal epithelium flap using diluted alcohol or forming a corneal epithelium flap using a corneal epithelium incision knife, followed by cutting the cornea via the application of a laser beam to a corneal stroma to correct vision. LASIK surgery, which has been developed to overcome the disadvantages of PRK surgery, has the advantages of reducing pain and turbidity of the cornea and shortening the vision recovery time. In LASIK surgery, however, complications, which may negatively affect vision, may occur during surgical operation and even after surgical operation. Moreover, in the case in which the thickness of the corneal stroma is too small after cutting of the cornea, corneal ectasia may occur, with the result that serious amblyopia may be caused. For this reason, lamellar keratoplasty has attracted attention, and LASEK surgery was developed by Massimo Camellin, an Italian ophthalmologist, in 1999.

LASEK surgery has advantages in that complications, such as wrinkling of a corneal flap, epithelial in-growth, and irregular flaps, do not occur, and the resistance to physical impacts is high, compared to LASIK surgery. In addition, LASEK surgery has advantages in that xerophthalmia does not occur and rapid recovery is possible, compared to LASIK surgery.

Small incision lenticule extraction (SMILE) surgery is surgery for incising some corneal stroma inside the surface of a cornea using a laser to correct abnormal refraction. In SMILE surgery, a piece of the corneal stroma that needs to be corrected is accurately cut in the shape of a lens in accordance with a desired amount of correction in the cornea using a precise VisuMax femtosecond laser, and then the cut piece of the corneal stroma is removed through an incision part formed in the cornea without forming a corneal flap, unlike laser in-situ keratomileusis (LASIK) and laser assisted sub-epithelial keratomileusis (LASEK). Consequently, SMILE surgery has effects in that damage to surrounding tissues is reduced, high precision is achieved, and the risk of side effects, such as myopic regression, congestion, and xerophthalmia, is remarkably reduced.

The above-described kinds of surgery are regarded as the same kind of surgery even though various kinds of lasers are used and various names are used depending on the manufacturer.

Hereinafter, SMILE surgery will be described in brief. A laser beam from a femtosecond laser is transmitted through the surface of a cornea and is applied to a corneal stroma, and a lenticule, which is a piece of the corneal stroma, is cut from the corneal stroma in the shape of a lens in accordance with a desired amount of correction. Afterwards, an incision part is formed in the cornea using the laser beam, and the cut lenticule is removed out of the cornea through the incision part.

The incision part includes an incision entrance formed in the surface of the cornea and an incision tunnel extending from the incision entrance toward the lenticule. The lenticule is removed out of the cornea through the incision tunnel and the incision entrance.

Meanwhile, when the lenticule is cut from the corneal stroma using the laser beam, tissue bridges, which are uncut regions, are formed between spots of the laser beam, since the spots of the laser beam are circular.

Even in the case in which the lenticule is three-dimensionally cut from the corneal stroma using the laser beam, therefore, a flap surface, which is a separated upper surface of the lenticule, and a lenticular surface, which is a separated lower surface of the lenticule, are not completely separated from the corneal stroma due to the tissue bridges. For this reason, a separation tool, such a long needle, is inserted through the incision entrance and the incision tunnel such that the separation tool moves along the flap surface and the lenticular surface to completely separate the lenticule from the corneal stroma, after which a picking tool, such as a pair of tweezers, is inserted through the incision entrance and the incision tunnel to remove the lenticule, to the outside through the incision entrance while holding the lenticule.

In the conventional art, however, the incision entrance and the incision tunnel are incised about 2 mm to 4 mm in the horizontal direction thereof. When the separation tool is inserted into the center of the cornea through the incision entrance and the incision tunnel and is rotated along the lenticular surface and the flap surface in alternating directions to separate the lenticule, which is connected to the corneal stroma via the tissue bridges, from the corneal stroma, the left and right sides of the incision entrance and the incision tunnel contact the separation tool, with the result that external force is applied to the left and right sides of the incision entrance and the incision tunnel.

In the case in which the incision entrance and the incision tunnel are formed to a length of about 2 mm in the horizontal direction in the course of SMILE surgery, the opposite sides of the incision entrance and the incision tunnel in the longitudinal direction may be easily torn by the external force generated as the result of motion of the separation tool. In the conventional art, therefore, the incision entrance and the incision tunnel are formed to a length of about 3.5 mm or more in the horizontal direction such that the opposite sides of the incision entrance and the incision tunnel in the longitudinal direction are prevented from being easily torn.

In addition, the higher the abnormal refraction, the thicker the lenticule that must be cut from the corneal stroma. When the incision part is formed to a length of 2 mm or less in the horizontal direction, and the lenticule is then separated from the corneal stroma while the separation tool is being carefully moved, the opposite sides of the incision entrance and the incision tunnel in the longitudinal direction are torn, because the size of the lenticule to be withdrawn through the incision tunnel and the incision entrance is too large.

However, the risk of corneal infection can be reduced, medical treatment time can be reduced, and tissues can be stabilized only when the length of the incision entrance and the incision tunnel in the horizontal direction is short. Therefore, there is a high necessity for a method that is capable of preventing the opposite sides of the incision entrance and the incision tunnel in the longitudinal direction from being easily torn while forming the incision entrance and the incision tunnel to a length of about 2 mm or less in the horizontal direction.

### [Disclosure]

### [Technical Problem]

Therefore, the present invention has been made in view of the above problems, and it is an object of the present invention to provide an incision part having a reinforcement part formed in a cornea to enable the removal of a lenticule cut in the course of vision correction surgery that is capable of effectively preventing opposite sides of the incision part from being torn while minimizing the length of the incision part, formed in the cornea to remove the lenticule, which is cut in the cornea.

It should be noted that objects of the present invention are not limited to the object of the present invention as mentioned above, and other unmentioned objects of the present invention will be clearly understood from the following description by those having ordinary skill in the technical field to which the present invention pertains.

### [Technical Solution]

In accordance with the present invention, the above and other objects can be accomplished by the provision of an incision part formed in a cornea to remove a lenticule cut in the course of vision correction surgery, the vision correction surgery for correcting abnormal refraction being performed by drawing a dense spiral toward the center of the cornea or outward from the center of the cornea while three-dimensionally changing the focal point of a laser beam to form a flap surface and a lenticular surface each having a continuous spiral incision line in a corneal stroma such that the flap surface and the lenticular surface are spaced apart from each other, making the outer circumference of the lenticular surface intersect the outer circumference of the flap surface inside the outer circumference of the flap surface to cut a three-dimensional lenticule from the corneal stroma, and removing the lenticule out of the cornea through an incision part formed in the cornea, wherein the incision part includes an incision entrance formed in the surface of the cornea adjacent to the outer circumference of the flap surface, the incision entrance being incised by the laser beam, and an incision tunnel extending from the incision entrance toward the corneal stroma, the tip of the incision tunnel being connected to the flap surface, and wherein the tip of the incision tunnel is connected to an incision line of the flap surface, and reinforcement parts, which are regions to which no laser beam is applied, are formed on opposite sides of the tip of the incision tunnel in the incision line of the flap surface.

The incision entrance and the incision tunnel may be incised by 0.5 mm to 2 mm or less in the horizontal direction thereof.

The distance between protrusions formed on the reinforcement parts may be equal to or greater than the length of the tip of the incision tunnel.

The tip of the incision tunnel may be connected to a portion of the incision line of the flap surface that is located at the outer circumference of the flap surface.

The tip of the incision tunnel may be connected to a portion of the incision line of the flap surface that is located at a point closer to the center of the flap surface than to the outer circumference of the flap surface.

The incision entrance may be formed in the surface of the cornea perpendicularly upward from the outer circumference of the flap surface.

The incision entrance may be formed in the surface of the cornea at an acute or obtuse angle relative to the outer circumference of the flap surface.

Each of the reinforcement parts may be semicircular or semielliptical.

Each of the reinforcement parts may be circular or elliptical.

Each of the reinforcement parts may be provided at a portion thereof with a protrusion that faces the tip.

The incision part may include a plurality of incision parts formed on opposite sides of the flap surface with respect to the center of the flap surface in the surface of the cornea adjacent to the outer circumference of the flap surface.

### [Advantageous Effects]

The present invention has effects in that, in small incision lenticule extraction (SMILE) surgery, which is surgery for three-dimensionally incising some corneal stroma inside the surface of a cornea using a femtosecond laser to correct abnormal refraction, an incision part, formed in the cornea to remove a piece of the corneal stroma, i.e. a lenticule, cut in the corneal stroma, out of the cornea, has a minimum length, and the opposite sides of the incision part in the longitudinal direction are not easily torn, thereby reducing the risk of corneal infection due to excessive length of the incision part, reducing the medical treatment time, and stabilizing tissues.

The effects of the present invention are naturally exhibited from the specification of the present invention irrespective of whether the inventors recognize the effects of the present invention. Consequently, the effects of the present invention are some effects of the present invention based on the specification of the present invention, and do not include all effects that the inventors have found or actually exist.

In addition, the effects of the present invention are further recognized through the specification of the present invention. Although not described clearly, any effects that can be recognized by those skilled in the art to which the present invention pertains from the specification of the present invention may be included in the effects of the present invention.

### [Description of Drawings]

The above and other objects, features and other advantages of the present invention will be more clearly understood from the following detailed description taken in conjunction with the accompanying drawings, in which:
FIG. 1A is a plan view of a cornea of an eyeball illustrating a lenticule and an incision part having a reinforcement part according to an embodiment of the present invention;
FIG. 1B is a sectional view of FIG. 1A;
FIG. 2A is a plan view of a cornea of an eyeball illustrating a lenticule and an incision part having a reinforcement part according to another embodiment of the present invention;
FIG. 2B is a sectional view of FIG. 2A;
FIG. 3 is a plan view of a cornea of an eyeball illustrating a lenticule and an incision part having a reinforcement part according to another embodiment of the present invention;
FIG. 4 is a plan view of a cornea of an eyeball illustrating a lenticule and an incision part having a reinforcement part according to a further embodiment of the present invention;
FIG. 5A is a plan view illustrating a process of separating a flap surface and a lenticular surface of a lenticule from a corneal stroma using a separation tool in accordance with an embodiment of the present invention; and
FIG. 5B is a plan view illustrating a process of separating a flap surface and a lenticular surface of a lenticule from a corneal stroma using a separation tool in accordance with another embodiment of the present invention.

### [Best Mode]

Reference will now be made in detail to the preferred embodiments, examples of which are illustrated in the accompanying drawings.

It should be noted herein that these embodiments are given only for illustrative purposes such that the present invention can be easily embodied by a person having ordinary skill in the art to which the present invention pertains, and the technical idea and category of the present invention are not limited thereto.

In the drawings, sizes and shapes of elements may be exaggerated for convenience and clarity of description. In addition, terms specially defined in consideration of the construction and operation of the present invention may vary depending upon intentions of users or operators or usual practices. The definition of such terms must be made based on the disclosure of the present invention.

An incision part formed in a cornea in order to remove a lenticule cut in a corneal stroma in the course of vision correction surgery according to the present invention mainly includes an incision entrance formed in the surface of the cornea and an incision tunnel formed so as to extend from the incision entrance into the corneal stroma, in which the lenticule is located, the tip of the incision tunnel being connected to a flap surface. Hereinafter, the incision part according to the present invention will be described in detail with reference to the accompanying drawings.

Before describing the incision part according to the present invention, the principle by which corneal tissues are cut will be described in brief. A femtosecond laser applies a pulse type laser beam to corneal tissues, and the corneal tissues are changed into gas plasma, from which micro-scale air bubbles are generated. A desired amount of corneal tissue is accurately cut as the result of continuous application of the laser beam. The laser beam radiated from the femtosecond laser is transmitted through the surface of the cornea and cuts a lenticule, which is a piece of the corneal stroma, in accordance with a desired amount of correction, thereby correcting imperfect vision, such as hyperopia, astigmatism, myopia, presbyopia, or mixed astigmatism, which is called small incision lenticule extraction (SMILE) surgery.

SMILE surgery may also be performed in the same manner using other lasers having similar effects.

Spots of the laser beam may form an optical penetration part in the corneal stroma. Plasma bubbles are generated as the result of such penetration, whereby a lenticule, a piece of the corneal stroma, is separated from the corneal stroma.

In order to cut the lenticule, the focal point of the laser beam is guided to a target in the cornea from below a corneal epithelium and a Bowman's membrane and above a Descemet's membrane and a corneal endothelium. To this end, the femtosecond laser has a tool for changing the focal point of the laser beam, a detailed description of which will be omitted.

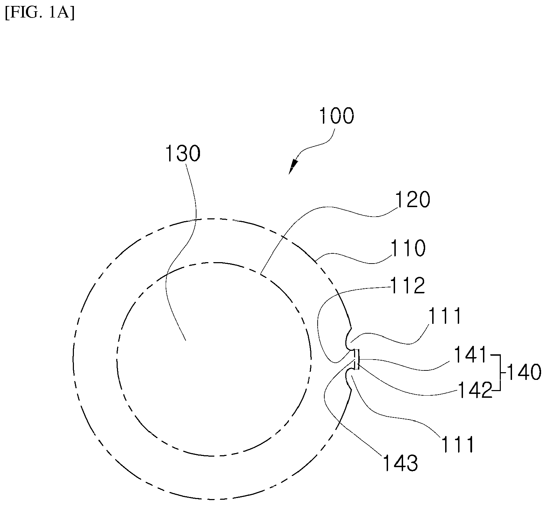

As illustrated in FIGS. 1A, 1B, 2A, 2B, 3, and 4, a laser beam is radiated into a corneal stroma 150 toward the center of a cornea 100 or outward from the center of the cornea 100 in a spiral fashion in the state in which the focal point of the laser beam is changed such that a flap surface 110, which is a separated upper surface that can be separated from the upper part of the corneal stroma 150, and a lenticular surface 120, which is a separated lower surface that can be separated from the lower part of the corneal stroma 150 while having the same central point as the flap surface 110, are formed in the state of being spaced apart from each other in the upward and downward direction. The flap surface 110 is formed so as to be larger than the lenticular surface 120. The outer circumference of the lenticular surface 120 intersects a point inside the outer circumference of the flap surface 110 that faces the center of the flap surface 110. As a result, a three-dimensional lenticule 130 is cut from the corneal stroma 150.

The outer circumference of the lenticular surface 120 may be spaced apart from the outer circumference of the flap surface 110 without intersecting a point inside the outer circumference of the flap surface 110 that faces the center of the flap surface 110. At this time, the outer circumference of the lenticular surface 120 may be connected to a point inside the outer circumference of the flap surface 110 that faces the center of the flap surface 110 through an incision surface (not shown) formed by the application of a laser beam, the focal point of which has been changed such that another three-dimensional lenticule, other than a lens type lenticule, may be cut from the corneal stroma 150.

The lenticule 130 cut in the corneal stroma 150 is removed out of the cornea 100.

In order to remove the lenticule 130 out of the cornea 100, an incision part 140, which includes an incision entrance 141 and an incision tunnel 142, is formed in the cornea 100 such that a separation tool 200 for completely separating the lenticule 130 from the corneal stroma 150 or a picking tool for removing the completely separated lenticule 130 out of the cornea 100 can be inserted into or withdrawn from the cornea 100.

The incision entrance 141 and the incision tunnel 142, which constitute the incision part 140, will be described with reference to FIGS. 1A, 1B, 2A, 2B, 3, and 4.

The incision entrance 141 is formed by applying a laser beam to the surface of the cornea 100 in a curved fashion in the horizontal direction using a femtosecond laser. In general, the incision entrance 141 has a horizontal length of about 2 mm to 4 mm and a vertical width of about 0.11 mm.

In the case in which reinforcement parts 111, a description of which will follow, are provided, the incision entrance 141 may have a horizontal length of about 0.5 mm to 2 mm or less.

In an example, the incision entrance 141 may be formed in the surface of the cornea 100 perpendicularly upward from the outer circumference of the flap surface 110, formed in the corneal stroma 150.

In another example, the incision entrance 141 may be formed in the surface of the cornea 100 at an acute or obtuse angle relative to the outer circumference of the flap surface 110, which is formed in the corneal stroma 150.

The incision entrance 141 may be formed at the top, the bottom, the left, and the right of the flap surface 110. In addition, the incision entrance 141 may be formed at various radial positions in the flap surface 110. The position of the incision entrance 141 may be changed depending on the direction in which an operator performs a surgical operation or the position of an eyeball fixing device.

A plurality of incision entrances 141 may be formed at the top, the bottom, the left, and the right of the flap surface 110 and at various radial positions of the flap surface 110.

The incision tunnel 142 is a path that extends from the incision entrance 141 to the lenticule 130 in the corneal stroma 150. The separation tool 200 may be inserted into the cornea 100 through the incision entrance 141 and the incision tunnel 142 to completely separately the lenticule 130 from the corneal stroma 150. The separated lenticule 130 may be removed out of the cornea 100 through the incision tunnel 142 and the incision entrance 141 using the picking tool.

The incision tunnel 142 is formed to a predetermined depth in the course of forming the incision tunnel 142 in the surface of the cornea 100 via the application of a laser beam. The laser beam is applied into the corneal stroma 150, in which the lenticule 130 is located. Preferably, a tip 143 of the incision tunnel 142 that faces the interior of the corneal stroma 150 is connected to the flap surface 110.

The incision tunnel 142 may be formed by an incision tool that is inserted into the corneal stroma 150, in which the lenticule 130 is located, through the incision entrance 141, after the incision entrance 141 is formed. The tip 143 of the incision tunnel 142 may be connected to the flap surface 110.

The incision tunnel 142 has a length equivalent to the horizontal length of the incision entrance 141. In addition, the incision tunnel 142 has a width equivalent to the vertical width of the incision entrance 141.

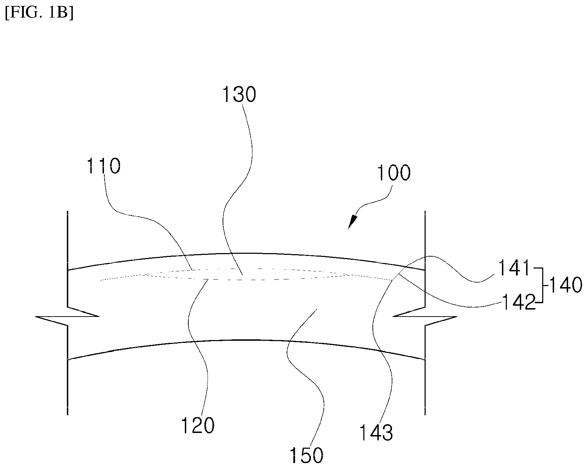

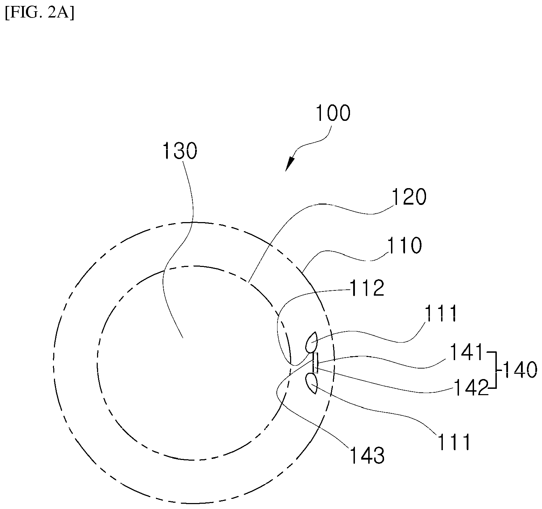

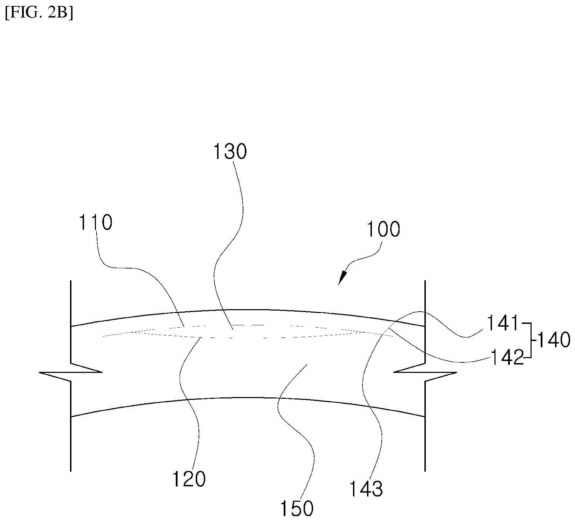

When the tip 143 of the incision tunnel 142 is connected to the flap surface 110, the tip 143 of the incision tunnel 142 may be connected to the outer circumference of the flap surface 110, as illustrated in FIG. 1B. Alternatively, the tip 143 of the incision tunnel 142 may be connected to a portion of the flap surface that is located at a point closer to the center of the flap surface than to the outer circumference of the flap surface, as illustrated in FIG. 2B.

Consequently, the incision tunnel 142 may be variously deformed depending on the position of the tip 143 of the incision tunnel 142 and the position of the incision entrance 141.

That is, the tip 143 of the incision tunnel 142 may be connected to the outer circumference of the flap surface 110, and the incision entrance 141 may be formed in the surface of the cornea 100 perpendicularly upward from the outer circumference of the flap surface 110, whereby the incision part 140 may be formed.

In addition, the tip 143 of the incision tunnel 142 may be connected to the outer circumference of the flap surface 110, and the incision entrance 141 may be formed in the surface of the cornea 100 at an acute or obtuse angle relative to the outer circumference of the flap surface 110, whereby the incision part 140 may be formed.

Furthermore, the tip 143 of the incision tunnel 142 may be connected to a point inside the outer circumference of the flap surface 110 that faces the center of the flap surface 110, and the incision entrance 141 may be formed in the surface of the cornea 100 perpendicularly upward from the outer circumference of the flap surface 110, whereby the incision part 140 may be formed.

Moreover, the tip 143 of the incision tunnel 142 may be connected to a point inside the outer circumference of the flap surface 110 that faces the center of the flap surface 110, and the incision entrance 141 may be formed in the surface of the cornea 100 at an acute or obtuse angle relative to the outer circumference of the flap surface 110, whereby the incision part 140 may be formed.

Meanwhile, as previously described, a laser beam is continuously applied outward from the center of the cornea 100 in a dense spiral fashion to form an incision line including a plurality of successive spots. The flap surface 110 may be incised from the corneal stroma 150 by the incision line.

Consequently, the connection between the tip 143 of the incision tunnel 142 and the outer circumference of the flap surface 110 means that the tip 143 of the incision tunnel 142 is connected to the last region of the incision line constituting the outer circumference of the flap surface 110. In addition, the connection between the tip 143 of the incision tunnel 142 and a point inside the outer circumference of the flap surface 110 that faces the center of the flap surface 110 means that the tip 143 of the incision tunnel 142 is connected to any one region of the incision line inside the outer circumference of the flap surface 110 that faces the center of the flap surface 110.

Laser beam spots are applied in a dense spiral fashion to incise the flap surface 110, which is the separated upper surface between the corneal stroma 150 and the lenticule 130, and the lenticular surface 120, which is the separated lower surface between the corneal stroma 150 and the lenticule 130, whereby the three-dimensional lenticule 130 is incised from the corneal stroma 150. However, the lenticule 130 is not completely separated from the corneal stroma 150 due to tissue bridges, which are regions between the respective laser beam spots.

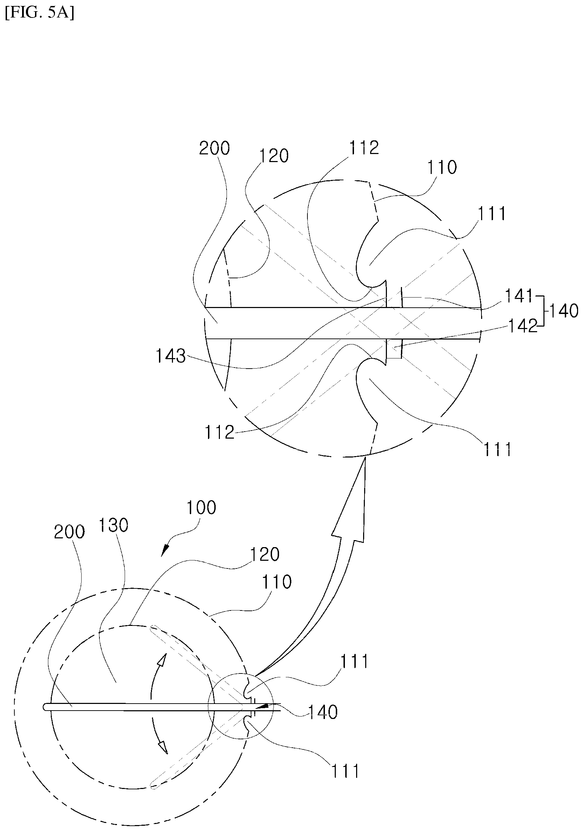

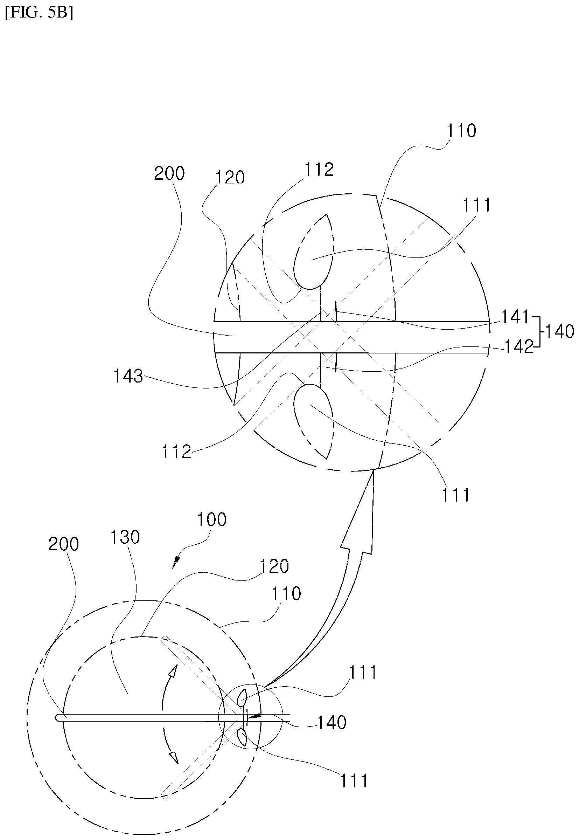

For this reason, as illustrated in FIGS. 5A and 5B, the separation tool 200, such as a needle, is inserted through the incision entrance 141 and the incision tunnel 142. The separation tool 200 entirely passes through the lenticular surface 120 and, in addition, entirely passes through the flap surface 110. As a result, the tissue bridges are cut, whereby the lenticule 130 is completely separated from the corneal stroma 150. Afterwards, the separation tool 200 is withdrawn, and the picking tool, such as a pair of tweezers, is inserted through the incision entrance 141 and the incision tunnel 142 to remove the lenticule 130, which has been completely separated from the corneal stroma 150, to the outside through the incision tunnel 142 and the incision entrance 141.

When the separation tool 200, which has been inserted through the incision entrance 141 and the incision tunnel 142, cuts the tissue bridges while moving leftward and rightward along the lenticular surface 120 and the flap surface 110, external force generated by the motion of the separation tool 200 is applied to the insides of the incision entrance 141 and the incision tunnel 142 in the longitudinal direction thereof.

When external force is applied to the insides of the incision entrance 141 and the incision tunnel 142 in the longitudinal direction thereof as the result of the motion of the separation tool 200, the tip 143 of the incision tunnel 142 is located in the incision line constituted by the laser beam spots applied in a dense spiral fashion. Consequently, the incision tunnel 142 cannot structurally withstand the external force applied to the insides of the incision tunnel 142 in the longitudinal direction thereof. As a result, the separation tool 200 easily moves along the incision line through the insides of the incision tunnel 142, whereby the insides of the incision tunnel 142 in the longitudinal direction thereof are torn, which affects the insides of the incision entrance 141 in the longitudinal direction thereof. Consequently, the incision entrance 141 is also torn.

In the case in which the incision entrance 141 and the incision tunnel 142 are torn, as described above, the risk of corneal infection is increased, the medical treatment time is increased, and tissues are not stabilized.

The present invention is characterized in that reinforcement parts 111, which are regions that are not incised, are provided at portions of the incision lines located on opposite sides of the tip 143 of the incision tunnel 142 in order to prevent the insides of the incision entrance 141 and the incision tunnel 142 in the longitudinal direction thereof from being easily torn by the separation tool 200.

In order to form the reinforcement parts 111, when the flap surface 110 is formed by a dense spiral incision line formed as the result of application of a laser beam, predetermined shaped regions to which the laser beam is not applied are formed on opposite sides of the tip 143 of the incision tunnel 132 in the incision line of the flap surface 110 connected to the tip 143 of the incision tunnel 142.

When the separation tool 200, which has been inserted through the incision entrance 141 and the incision tunnel 142, is rotated leftward and rightward, therefore, the reinforcement parts 111 sufficiently withstand the external force generated by the rotation of the separation tool 200 even though the external force generated by the rotation of the separation tool 200 is applied to the insides of the incision tunnel 142 in the longitudinal direction thereof, whereby it is possible to prevent the insides of the incision tunnel 142 in the longitudinal direction thereof from being easily torn.

The reinforcement parts 111 may be configured by modifying a program that governs whether to apply a laser beam using a femtosecond laser such that no laser beam is applied to regions corresponding to the shape of the reinforcement parts 111 to be formed when the laser beam is applied while drawing a dense spiral line including a plurality of successive spots in order to form the flap surface 110. The shape of the reinforcement parts 111 may be variously changed through various application of the program.

Alternatively, it is possible to use a method of placing laser beam blocking members having a shape corresponding to the shape of the reinforcement parts 111 on the incision lines located on the opposite sides of the tip of the incision tunnel 142, which is formed by the laser beam, such that no laser beam is applied to the corneal stroma.

In the case in which the insides of the incision tunnel 142 in the longitudinal direction thereof are prevented from being torn by the provision of the reinforcement parts 111, as described above, the insides of the incision entrance 141 in the longitudinal direction thereof may also be prevented from being torn. Consequently, the incision entrance 141 and the incision tunnel 142 may be minimally incised by about 0.5 mm to 2 mm or less in the horizontal direction thereof.

Meanwhile, the reinforcement parts 111 are formed at opposite outsides of the tip 143 of the incision tunnel 142 in the incision line of the flap surface 110, in which the tip 143 of the incision tunnel 142 is located. In the case in which the tip 143 of the incision tunnel 142 is located in the last region of the incision line constituting the outer circumference of the flap surface 110, as illustrated in FIG. 1A, the reinforcement parts 111 are formed at opposite sides of the tip 143 of the incision tunnel 142 in the state in which the reinforcement parts 111 are symmetric with respect to the tip 143 of the incision tunnel 142. At this time, each of the reinforcement parts 111 may be semicircular or semielliptical. Alternatively, each of the reinforcement parts 111 may have an irregular non-circular shape. In addition, each of the reinforcement parts 111 may have various shapes, such as a semi-conical shape and a half-water drop shape.

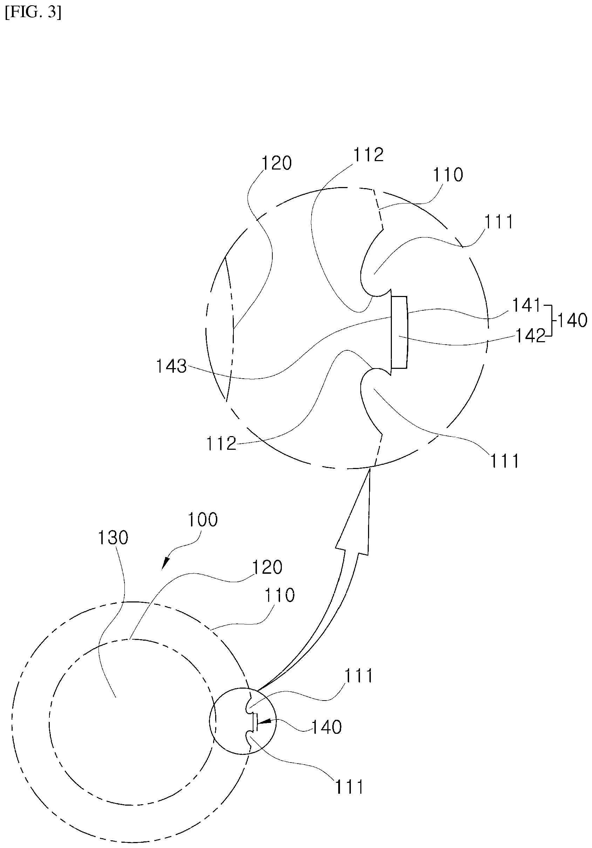

Preferably, as illustrated in FIG. 3, a portion of each of the reinforcement parts 111 that is adjacent to the tip 143 of the incision tunnel 142 may be provided with a protrusion 112 that protrudes toward the tip 143 of the incision tunnel 142, and the protrusion 112 may be configured so as not to interfere with the incision tunnel 142.

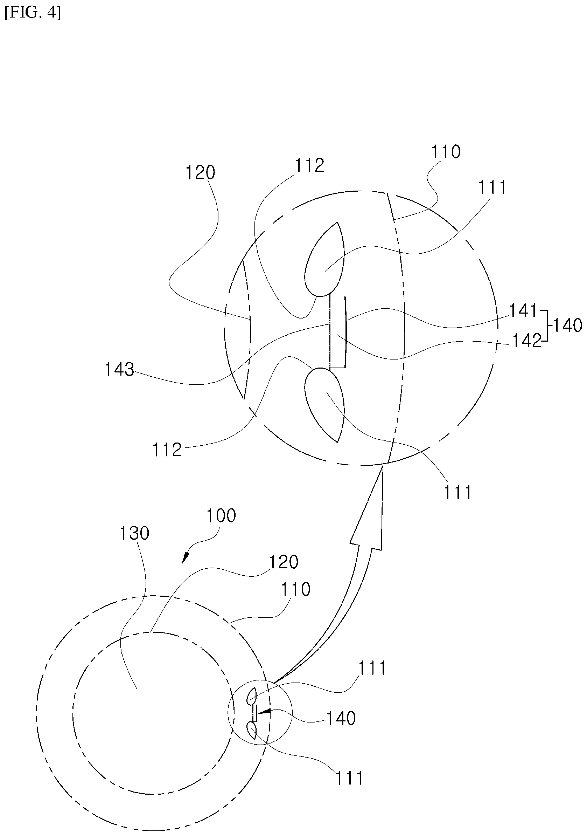

In addition, in the case in which the tip 143 of the incision tunnel 142 may be located in a portion of the incision line that is located at a point closer to the center of the flap surface 110 than to the outer circumference of the flap surface 110, each of the reinforcement parts 111 may be semicircular or semielliptical. Alternatively, each of the reinforcement parts 111 may have an irregular non-circular shape. In addition, each of the reinforcement parts 111 may have various shapes, such as a semi-conical shape and a half-water drop shape. In this case, as illustrated in FIG. 4, a portion of each of the reinforcement parts 111 that is adjacent to the tip 143 of the incision tunnel 142 may be provided with a protrusion 112 that protrudes toward the tip 143 of the incision tunnel 142, and the protrusion 112 may be configured so as not to interfere with the incision tunnel 142.

Hereinafter, the main process of SMILE surgery using the incision part 140 having the reinforcement parts 111 will be described briefly.

First, a patient, who has been partially anesthetized, is placed on a bed, at which a femtosecond laser is installed, and triple centration is performed in consideration of an error between a pupil axis and a visual axis.

Centration is a technique of performing a surgical operation while being aligned with the center of the cornea, which may improve the accuracy of the surgical operation and may prevent blurring, photophobia, double vision, and amblyopia.

Triple centration may include a step of setting centration using a marking pen for surgical operation in consideration of an error between a pupil center and a visual axis, a step of aligning the center of the femtosecond laser with the marked area, and a step of applying a laser beam to the center of the marked area using the femtosecond laser.

After the triple centration is completed, a laser beam from the femtosecond laser, which is programmed in accordance with a desired amount of correction, is applied to the cornea 100. A plurality of spots is continuously formed while a spiral line is drawn from the outside of the cornea 100 to the center of the cornea 100 in order to form a lenticular surface 120, which will be separated from the corneal stroma 150, first.

Subsequently, as illustrated in FIGS. 1B and 2B, the focal point of the laser beam applied by the femtosecond laser is changed, with the result that the focal point of the laser beam is formed on the lenticular surface 120, and a plurality of spots is continuously formed while drawing a spiral line from the center of the cornea 100 to the outside of the cornea 100 in order to form a flap surface 110, which will be separated from the corneal stroma 150. At this time, as illustrated in FIGS. 1A and 1B, the flap surface 110 is formed so as to be larger than the lenticular surface 120. The outer circumference of the lenticular surface 120 intersects the outer circumference of the flap surface 110 inside the outer circumference of the flap surface 110 in order to cut a three-dimensional lenticule 130, which is constituted by the upper flap surface 110 and the lower lenticular surface 120, from the corneal stroma 150, as illustrated in FIGS. 1B and 2B.

In the case in which a region at which reinforcement parts 111 are formed, for example the incision tunnel 142 of the incision part 140, is connected to the incision line located at the outer circumference of the flap surface 110, as illustrated in FIG. 3, in the process of forming the flap surface 110 as the result of the application of the laser beam, reinforcement parts 111, which are regions to which no laser beam is applied, are formed on opposite sides of the tip 143 of the incision tunnel 142 in the incision line in consideration of the position of the tip 143 of the incision tunnel 142 and the shape of the reinforcement parts 111 to be formed when the incision line located at the outer circumference of the flap surface 110 is formed.

After the lenticule 130 is completely separated from the corneal stroma 150, an incision entrance 141, having a curved horizontal length, and an incision tunnel 142, having the same horizontal length as the incision entrance 141 and communicating with the incision line of the flap surface between the reinforcement parts 111, are formed at the surface of the cornea 100 adjacent to the outer circumference of the flap surface 110 in order to remove the lenticule 130 out of the cornea 100.

When the reinforcement parts 111 are formed at the flap surface 110, the separation tool 200 comes into contact with the protrusions 112 of the reinforcement parts 111 during the rotation or movement of the separation tool 200 even though the incision entrance 141 and the incision tunnel 142 are formed to a length of about 2 mm, whereby it is possible to prevent the incision entrance 141 and the incision tunnel 142 from being torn. The length of the incision entrance 141 and the incision tunnel 142 is not necessarily limited to about 2 mm. The incision entrance 141 and the incision tunnel 142 may have a minimum length sufficient to allow the separation tool 200 to be inserted therethrough. For example, the incision entrance 141 and the incision tunnel 142 may be incised by about 0.5 mm to 2 mm.

After the reinforcement parts 111, the incision tunnel 142, and the incision entrance 141 are formed, the separation tool 200 is inserted through the incision entrance 141 and the incision tunnel 142 in order to completely separate the lenticular surface 120 and the flap surface 110, which have not been completely separated from the corneal stroma 150 due to tissue bridges, from the corneal stroma 150. As illustrated in FIGS. 5A and 5B, the separation tool 200 is inserted into the incision line constituting the outer circumference of the flap surface 110 through the incision entrance 141 and the incision tunnel 142, and is then rotated along the lenticular surface 120 in alternating directions in order to completely separate the lenticular surface 120 from the corneal stroma 150.

At this time, the surgical operation may be performed without using an eyeball fixing device in order to minimize bleeding, congestion, and pain.

After the lenticular surface 120 is completely separated from the corneal stroma 150, the separation tool 200 may be completely withdrawn from the incision entrance 141 and may then be inserted through the incision entrance 141 to separate the flap surface 110 from the corneal stroma 150. Alternatively, the separation tool 200 may be minutely lifted in the incision tunnel 142 without completely withdrawing the separation tool 200 from the incision entrance 141 such that the separation tool 200 is directly moved to the flap surface 110, after which the separation tool 200 may be rotated along the flap surface 110 in alternating directions in order to completely separate the flap surface 110 from the corneal stroma 150. In this case, the medical treatment time may be minimized.

When external force generated by the motion of the separation tool 200 is applied to the insides of the incision tunnel 142 in the longitudinal direction thereof in the course of rotating the separation tool 200, inserted through the incision entrance 141 and the incision tunnel 142, in alternating directions in order to completely separate the lenticular surface 120 and the flap surface 110 from the corneal stroma 150, the separation tool 200 comes into contact with the protrusions 112 of the reinforcement parts 111 first, whereby it is possible to prevent the insides of the incision tunnel 142 in the longitudinal direction thereof from being torn. In addition, it is also possible to prevent the insides of the incision entrance 141 in the longitudinal direction thereof from being torn.

Since the strength of the insides of the incision tunnel 142 in the longitudinal direction thereof is increased by the provision of the reinforcement parts, therefore, it is possible to prevent the incision entrance 141 and the incision tunnel 142 from being torn by the separation tool 200 even though the incision length of the incision entrance 141 and the incision tunnel 142 in the horizontal direction thereof is minimized to 0.5 mm to 2 mm. The case in which the incision length of the incision entrance 141 and the incision tunnel 142 is minimized is advantageous in reducing the risk of corneal infection, reducing the medical treatment time, and stabilizing tissues.

After the lenticule 130 is completely separated from the corneal stroma 150 using the separation tool 200, a picking tool is inserted into the corneal stroma 150, in which the lenticule 130 is located, through the incision entrance 141 and the incision tunnel 142 in order to pick the lenticule 130 and to remove the picked lenticule 130 out of the corneal stroma 150 through the incision entrance 141. Afterwards, eye drops are supplied through the incision entrance 141, and then suspended matters are removed using the separation tool.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

### [Industrial Applicability]

The present invention is configured to have a structure in which, when a lenticule, which is constituted by a lenticular surface and a flap surface, is cut in a corneal stroma by applying a laser beam while three-dimensionally changing the focal point of the laser beam and the cut lenticule is removed out of a cornea through an incision part, which includes an incision entrance and an incision tunnel, reinforcement parts are provided at the connections of the incision tunnel and the flap surface such that the incision entrance and the incision tunnel are prevented from being torn by external force, generated by the motion of a separation tool inserted into the incision tunnel, by the provision of the reinforcement parts. Consequently, the incision length of the incision entrance and the incision tunnel in the horizontal direction thereof is minimized to 0.5 mm to 2 mm, thereby reducing the risk of corneal infection, reducing the medical treatment time, which reduces the inconvenience to a patient, and rapidly regenerating and stabilizing tissues, which improves the surgical operation and the patient's satisfaction. Consequently, the present invention is very useful from the aspect of industrial applicability.

## Claims

1. An incision part formed in a cornea to remove a lenticule cut in the course of vision correction surgery, the vision correction surgery for correcting abnormal refraction being performed by drawing a dense spiral toward a center of the cornea or outward from the center of the cornea while three-dimensionally changing a focal point of a laser beam to form a flap surface and a lenticular surface each having a continuous spiral incision line in a corneal stroma such that the flap surface and the lenticular surface are spaced apart from each other, making an outer circumference of the lenticular surface intersect an outer circumference of the flap surface inside the outer circumference of the flap surface to cut a three-dimensional lenticule from the corneal stroma, and removing the lenticule out of the cornea through an incision part formed in the cornea, wherein the incision part comprises:
an incision entrance formed in a surface of the cornea adjacent to the outer circumference of the flap surface, the incision entrance being incised by the laser beam; and
an incision tunnel extending from the incision entrance toward the corneal stroma, a tip of the incision tunnel being connected to the flap surface, and wherein
the tip of the incision tunnel is connected to an incision line of the flap surface, and reinforcement parts, which are regions to which no laser beam is applied, are formed on opposite sides of the tip of the incision tunnel in the incision line of the flap surface.

2. The incision part according to claim 1, wherein the incision entrance and the incision tunnel are incised by 0.5 mm to 2 mm or less in a horizontal direction thereof.

3. The incision part according to claim 2, wherein a distance between protrusions formed on the reinforcement parts is equal to or greater than a length of the tip of the incision tunnel.

4. The incision part according to claim 1, wherein the tip of the incision tunnel is connected to a portion of the incision line of the flap surface that is located at the outer circumference of the flap surface.

5. The incision part according to claim 1, wherein the tip of the incision tunnel is connected to a portion of the incision line of the flap surface that is located at a point closer to a center of the flap surface than to the outer circumference of the flap surface.

6. The incision part according to claim 4 or 5, wherein the incision entrance is formed in the surface of the cornea perpendicularly upward from the outer circumference of the flap surface.

7. The incision part according to claim 4 or 5, wherein the incision entrance is formed in the surface of the cornea at an acute or obtuse angle relative to the outer circumference of the flap surface.

8. The incision part according to claim 4, wherein each of the reinforcement parts is semicircular or semielliptical.

9. The incision part according to claim 5, wherein each of the reinforcement parts is circular or elliptical.

10. The incision part according to claim 8 or 9, wherein each of the reinforcement parts is provided at a portion thereof with a protrusion that faces the tip.

11. The incision part according to claim 1, wherein the incision part comprises a plurality of incision parts formed on opposite sides of the flap surface with respect to a center of the flap surface in the surface of the cornea adjacent to the outer circumference of the flap surface.
